# EUROPÄISCHE PATENTSCHRIFT

(11) **EP 2 228 643 B1**
(45) Veröffentlichungstag und Bekanntmachung des Hinweises auf die Patenterteilung: **14.12.2016**
(21) Anmeldenummer: 10005996.3
(22) Anmeldetag: 14.01.2008
(51) Int. Cl.: C21C 1/08, F27D 21/00, C21C 7/00

(54) **Verfahren zum Beeinflussen der maximalen Ausdehnung von Gusseisen**
Method for influencing the elongation of cast iron
Procédé destiné à influencer l'élongation de fontes

(30) Priorität: 22.01.2007 DE 102007004147
(43) Veröffentlichungstag der Anmeldung: 15.09.2010
(62) Teilanmeldung aus: 08707030.6
(73) Patentinhaber: Heraeus Electro-Nite International N.V., 3530 Houthalen (BE)
(72) Erfinder: Habets, Danny, 3600 Genk (BE)
(74) Vertreter: Heraeus IP

(56) Entgegenhaltungen:
- WO-A1-99/45156
- DE-A1- 2 923 236
- JP-A- 2006 063 396
- US-B1- 6 544 359

## Beschreibung

Die Erfindung betrifft ein Verfahren zur Beeinflussung der Eigenschaften von Gusseisen durch Zugabe von Magnesium zur Gusseisenschmelze.

Allgemein wird der freie Magnesiumgehalt in einer Gusseisenschmelze als bestimmender Faktor angesehen für die Bildung von sphäroidischem oder vermikularem Graphit im magnesiumbehandeltem Gusseisen. Die gegenwärtige Praxis zur Regelung der Produktion von duktilem Gusseisen besteht in der Bestimmung des Gesamt-Magnesiumgehaltes, das heißt, des freien und des gebundenen Magnesiums, mit Hilfe von spektrographisch untersuchten Proben. Diese Methode ergibt allerdings ein unvollständiges Bild, da der Gehalt an freiem Magnesium nicht bekannt ist und die Messung keine Information über die Sauerstoffaktivität ergibt. Die Sauerstoffaktivität, die im Gleichgewicht mit dem freien Magnesium ist, ist jedoch ein bestimmender Faktor in der Formierung der Graphitform. Das sogenannte duktile Gusseisen ist normaler Grauguss, der mit einem eine Kugel bildenden Zusatz behandelt ist, so dass der Hauptteil des graphitischen Kohlenstoffs im Gusseisen sogenannter Nodulargraphit (Knötchengraphit) oder kugelförmiger Graphit ist. Nodulargraphit in Gusseisen muss hinsichtlich Form, Größe und Teilchenzahl analysiert werden, da diese Kenngrößen die mechanischen Eigenschaften des Gusseisens beeinflussen. Eine visuelle Analyse ist komplex oder subjektiv, selbst bei teilautomatisierten Analysen. Messungen hierzu sind beispielsweise aus US 5,675,097 bekannt. In der DE 199 28 456A1 sind Messungen zur Bestimmung der räumlichen Struktur von Graphit in Gusseisen beschrieben, die auf eine Sauerstoffbestimmung basieren und die Nachteile visueller Methoden nicht aufweisen. Dadurch kann schneller reagiert werden und die gezielte Beeinflussung der Produktion erhöht die Ausbeute bzw. senkt den Ausschuss beim Gießen. Die Qualität des Gusseisens wird gut regelbar.

Weitere Beispiele zur Messung von Sauerstoff zur Kontrolle der Zusammensetzung von Mg behandeltem Gusseisen sind aus JP2006063396, US6,544,359 B1 und WO 99/45156 bekannt.

Der Erfolg der Magnesiumbehandlung in Gusseisen kann beispielsweise durch metallographische oder spektrographische Analysen von weißerstarrten Proben oder auch durch thermische Analysen erfolgen.

Allgemein wird reines Magnesium oder eine Magnesiumlegierung genutzt, um die Kugelform des Gusseisens zu fördern. Ein Teil des zugefügten Magnesiums entzieht dem Eisen Sauerstoff und Schwefel, der verbleibende Teil ist der sogenannte freie Magnesiumanteil, der die Sauerstoffaktivität regelt. Der freie Magnesiumgehalt in der Schmelze ist der bestimmende Faktor für die Nodularität des Gusseisens. Der freie Magnesiumanteil nimmt in der Schmelze im Laufe der Zeit ab, während die Sauerstoffaktivität steigt. Dies beeinflusst die Struktur und mechanischen Eigenschaften des Gusseisens.

Sensoren zur Bestimmung der Sauerstoffaktivität einer Metallschmelze sind beispielsweise aus DE 103 10 387 B3 bekannt. Hier ist ein Festelektrolytröhrchen offenbart, das an seiner äußeren Oberfläche eine Beschichtung aus einer Mischung aus Kalziumzirkonat und einem Fluorid aufweist, so dass beispielsweise in Eisenschmelzen die Messung der Konzentration von Schwefel, Silizium oder Kohlenstoff erfolgen kann.

Aufgabe der vorliegenden Erfindung ist es, ein Verfahren zur Regelung des Verfahrens anzugeben, mit dem die bisherige Technik verbessert wird, wobei die mechanischen Eigenschaften des Gusseisen bereits in der flüssigen Phase gezielt beeinflusst werden sollen.

Die Aufgabe wird gelöst durch die Merkmale des unabhängigen Anspruchs. Vorteilhafte Ausgestaltungen sind in dem Unteranspruch angegeben. Insbesondere ist das erfindungsgemäße Verfahren dadurch gekennzeichnet, dass der Sauerstoffgehalt der Gusseisenschmelze gemessen wird und dass der Gusseisenschmelze so lange Magnesium zugeführt wird, bis der Sauerstoffgehalt der Gusseisenschmelze bei einer Temperatur von 1.420C° als Referenztemperatur 0,08 bis 0,1 ppm beträgt. Da die Sauerstoffmessung genauer ist als die bisher mögliche Magnesiummessung (Magnesium liegt in der Schmelze als freies Magnesium und als gebundenes Magnesium vor, so dass eine genaue Erfassung nicht möglich ist), ist die Bestimmung der mechanischen Eigenschaften des Gusseisens genauer. Der Fachmann kann eine Korrelation zwischen der Existenz weniger großer Graphitpartikel bei niedrigem Sauerstoffgehalt einerseits und vieler kleiner Graphitpartikel bei höherem Sauerstoffgehalt andererseits feststellen und nutzen.

Damit ist eine Korrelation zu den mechanischen Eigenschaften, wie bereits in US 5,675,097 beschrieben, möglich, wie zum Beispiel hinsichtlich der Bruchfestigkeit, der Dehnung und des Widerstandes gegen Verformungen. Für Gusseisen hat es sich überraschend erwiesen, dass es eine maximale Dehnung dann aufweist, wenn die Magnesiumzugabe solange erfolgt, bis der Sauerstoffgehalt kleiner ist als 0,1 ppm, erfindungsgemäß zwischen 0,08 und 0,1 ppm. Bei niedrigerem oder höherem Sauerstoffgehalt nimmt die Dehnung des Gusseisens wieder ab. Vorteilhaft ist es, dass etwa 200 bis 750 ppm Magnesium zu der Gusseisenschmelze zugegeben werden, um den gewünschten Sauerstoffgehalt zu erreichen.

Ein Sensor zum Messen des Sauerstoffgehaltes in Gusseisenschmelzen mit einer elektrochemischen Messzelle, die ein Festelektrolytröhrchen aufweist ist dadurch gekennzeichnet, dass auf der nach außen gerichteten Oberfläche des Festelektrolytröhrchens eine Schicht aus Zirkondioxid aufgebracht ist. Insbesondere kann das Zirkondioxid der Schicht mit Kalziumoxid, Yttriumoxid und/oder Magnesiumoxid stabilisiert sein. Vorteilhaft ist es, dass die Schicht mit bis zu 30 Gew.-% Kalziumoxid, bis zu 25 Gew.-% Magnesiumoxid und/oder bis 52 Gew.-% Yttriumoxid stabilisiert ist. Insbesondere ist es vorteilhaft, dass die Schicht mit etwa 4 bis 6 Gew.-% Kalziumoxid stabilisiert ist. Vorteilhafterweise ist die Schicht des Sensors plasmagespritzt. Sie weist vorzugsweise eine Dicke von etwa 30 bis 50 µm, insbesondere etwa 40 µm auf. Das Festelektrolytröhrchen, auf welchem die Schicht angeordnet ist, ist vorzugsweise ein Zirkondioxidröhrchen, welches mit etwa 2 Gew.-% Magnesiumoxid stabilisiert sein kann.

Nachfolgend wird ein Ausführungsbeispiel der Erfindung anhand einer Zeichnung beschrieben. In der Zeichnung zeigt:
Figur 1 den Zusammenhang zwischen der Zahl der Graphitteilchen und dem Sauerstoffgehalt (Sauerstoffaktivität aO),
Figur 2 den Zusammenhang zwischen der relativen Ausdehnung und dem Sauerstoffgehalt,
Figur 3 einen Querschnitt durch einen Sensorkopf und
Figur 4 einen Teilschnitt durch eine weitere Ausführungsform des Sensors.

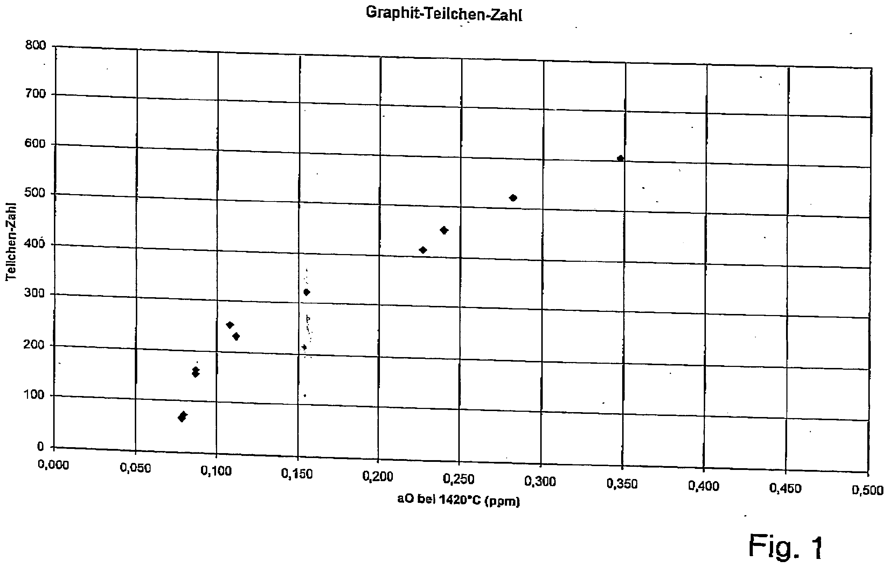

In Figur 1 ist zu sehen, dass die Zahl der Graphitteilchen mit zunehmendem Sauerstoffgehalt (Sauerstoffaktivität aO) steigt. Mit Hilfe der Regelung des Sauerstoffgehaltes über die Zugabe von Magnesium kann also die Zahl der Graphitteilchen eingestellt werden. Damit werden die Eigenschaften des Gusseisens bereits in der Schmelze gezielt beeinflusst. Eine maximale Nodularität tritt bei einer Sauerstoffaktivität zwischen etwa 0,10 und 0,12 ppm auf (gültig für 1.420°C). Fällt die Sauerstoffaktivität unter 0,10 ppm, reduziert sich die Nodularität.
Dies entspricht der bekannten Erfahrung aus der Gießereipraxis, dass ein zu hoher Magnesiumanteil negative Auswirkungen auf die Nodularität hat.

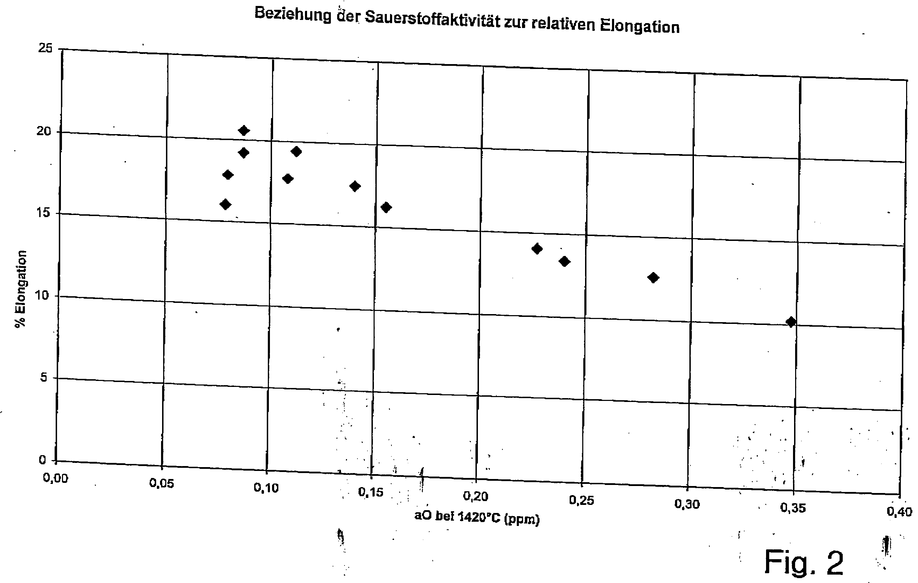

Figur 2 zeigt den Zusammenhang zwischen der relativen Ausdehnung des Gusseisens und dem Sauerstoffgehalt. Ein Maximum der Ausdehnung (Elongation) ist bei etwa 0,08 ppm erkennbar. Bei niedrigerer Sauerstoffaktivität ist die Dehnung geringfügig kleiner, vermutlich wegen der geringeren Nodularität. Übersteigt die Sauerstoffaktivität den optimalen Wert, kommt es zu einer stetigen Verringerung der Dehnung. Die Graphik zeigt, dass es möglich ist, durch die Einstellung des Sauerstoffgehaltes in der Gusseisenschmelze durch Zugabe von Magnesium die relative Ausdehnung des Gusseisens zu beeinflussen.

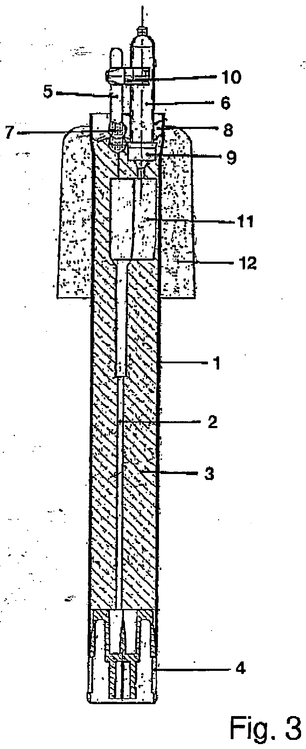

In Figur 3 ist ein Sensor dargestellt. In einem Metallrohr 1 sind die elektrischen Leitungen 2 (Cu/CuNi/Leiter) in einer Sandfüllung 3 angeordnet. Über das Verbindungsstück 4 werden die elektrischen Leitungen mit einer Lanze oder einem anderem Halter und des Weiteren mit einer Auswertereinheit verbunden. Das andere Ende der Leitungen 2 ist mit einem Thermoelement 5 und der elektrochemischen Messzelle 6 verbunden. Die elektrochemische Messzelle 6 weist ein Festelektrolytröhrchen (ZrO₂-Zelle) mit einem Stahlschockschild als äußere Hülle auf. Die ZrO₂-Zelle weist an ihrer äußeren Oberfläche eine Schicht aus Zirkondioxid auf, die mit 5 Gew.-% Kalziumoxid stabilisiert ist. Diese Schicht ist etwa 40 µm dick. In der Zeichnung ist sie nicht im Einzelnen dargestellt, da Festelektrolytröhrchen grundsätzlich bekannt sind.

Das Thermoelement 5 ist in einem Thermoelement-Dichtungszement 7 fixiert. Die Messzelle 6 ist ebenfalls in einem Zement 8 fixiert, ihr im Inneren des Sensors angeordnetes Ende ist mit einem Dichtungsstöpsel 9 geschlossen, durch den die elektrischen Kontakte herausgeführt sind. Die beiden Sensorelemente 5; 6 sind mittels eines Plastikclips 10 miteinander verbunden. Durch das thermisch isolierende Teil 11 hindurch werden die Leitungen durch das Innere des Metallrohres 1 geführt. An dem Eintauchen des Sensors ist ein Sandkörper 12 an der Außenseite des Metallrohres 1 angeordnet, um dieses zu schützen.

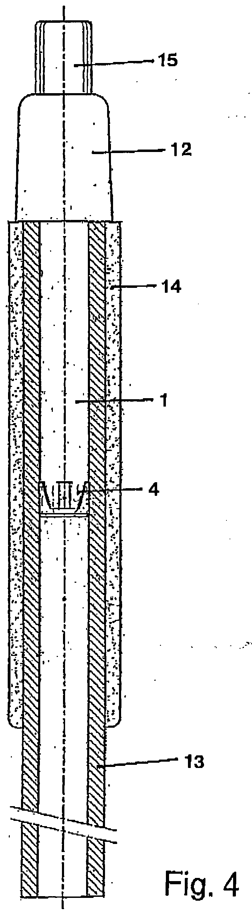

Figur 4 zeigt eine ähnliche Anordnung, bei der die Kontaktierung des Sensors im Trägerrohr 13 dargestellt ist. Das Trägerrohr 13 ist aus Pappe gebildet und an seiner vorderen, dem Sandkörper 12 zugewandten Seite von einem Spritzschutzrohr 14 umgeben, welches aus Gießereisand oder Zement gebildet ist.
Die Sensorelemente 5; 6 selbst sind zum Schutz beim Transport und beim Eintauchen in die Schmelze zunächst mit einer Metallkappe 15 umgeben, die beim bzw. nach dem Eintauchen des Sensors in die Metallschmelze schmilzt und die Sensorelemente 5; 6 freigibt.

Die Sensorelemente 5; 6 selbst sind zum Schutz beim Transport und beim Eintauchen in die Schmelze zunächst mit einer Metallkappe 15 umgeben, die beim bzw. nach dem Eintauchen des Sensors in die Metallschmelze schmilzt und die Sensorelemente 5; 6 freigibt.

## Patentansprüche

1. Verfahren zur Beeinflussung der Elongation von Gusseisen durch Zugabe von Magnesium zur Gusseisenschmelze, **dadurch gekennzeichnet, dass** der Sauerstoffgehalt der Gusseisenschmelze gemessen wird und dass der Gusseisenschmelze solange Magnesium zugeführt wird, bis der Sauerstoffgehalt der Gusseisenschmelze bei einer Temperatur von 1.420°C zwischen 0,08 und 0,1 ppm beträgt.

2. Verfahren nach Anspruch 1, **dadurch gekennzeichnet, dass** etwa 200 bis 750 ppm Magnesium zugegeben werden.

## Claims

1. Method for influencing the elongation of cast iron by adding magnesium to the molten cast iron, **characterised in that** the oxygen content of the molten cast iron is being measured and **in that** magnesium is being added to the molten cast iron until the oxygen content of the molten cast iron at a temperature of 1,420°C is between 0.08 and 0.1 ppm.

2. Method according to claim 1, **characterised in that** approximately 200 to 750 ppm magnesium are being added.

## Revendications

1. Procédé d'influencer l'élongation de fonte de fer par addition de magnésium à la masse fondue de fonte de fer, **caractérisé en ce que** la teneur en oxygène de la masse fondue de fonte de fer est mesurée et que du magnésium est ajouté à la masse fondue de fonte de fer jusqu'à ce que la teneur en oxygène de la masse fondue de fonte de fer soit comprise entre 0,08 et 0,1 ppm à une température de 1420°C.

2. Procédé selon la revendication 1, **caractérisé en ce qu'**environ 200 à 750 ppm de magnésium sont ajoutées.
